Europäisches Patentamt

European Patent Office

Office européen des brevets

(11) Veröffentlichungsnummer: **0 295 548 B1**

(12) **EUROPÄISCHE PATENTSCHRIFT**

(45) Veröffentlichungstag der Patentschrift: **18.03.92**

(21) Anmeldenummer: **88109084.9**

(22) Anmeldetag: **08.06.88**

(51) Int. Cl.⁵: **C07C  69/533**, C07C 67/333

(54) Verfahren zur Herstellung von Pentensäureestern aus Formylvaleriansäureestern.

(30) Priorität: **15.06.87 DE 3719937**

(43) Veröffentlichungstag der Anmeldung:
**21.12.88 Patentblatt  88/51**

(45) Bekanntmachung des Hinweises auf die
Patenterteilung:
**18.03.92 Patentblatt  92/12**

(84) Benannte Vertragsstaaten:
**BE CH DE ES FR GB IT LI NL**

(56) Entgegenhaltungen:
**DE-A- 1 917 244**
**DE-A- 2 425 654**
**US-A- 4 272 444**

**JOURNAL OF THE AMERICAN CHEMICAL SO-
CIETY; Band 90, Nr. 1, 3. Januar 1968, S.
99-107, American Chemical Society, Columbus, Ohio, USA; K. OHNO et al, "Organic
Syntheses by Means of Noble Metal Compounds. XXXV. Novel Decarbonylation Reactions of Aldehydes and Acyl Halides Using
Rhodium Complexes"**

**CHEMICAL ABSTRACTS, Band 90, Nr. 25, 18.
Juni 1979, Zusammenfassung Nr. 203499a,
Columbus, Ohio, USA; & CA-A-1048533**

(73) Patentinhaber: **BASF Aktiengesellschaft
Carl-Bosch-Strasse 38
W-6700 Ludwigshafen(DE)**

(72) Erfinder: **Merger, Franz, Dr.
Max-Slevogt-Strasse 25
W-6710 Frankenthal(DE)**
Erfinder: **Frank, Jürgen, Dr.
Hirschbrunnenweg 82
W-6830 Schwetzingen(DE)**
Erfinder: **Vagt, Uwe, Dr.
Paul-Neumann-Strasse 44
W-6720 Speyer(DE)**
Erfinder: **Fischer, Rolf, Dr.
Bergstrasse 98
W-6900 Heidelberg(DE)**

**Beschreibung**

Bei der Hydroformylierung von Pentensäureestern, wie sie in den europäischen Patentanmeldungen 31 100 und 125 567 beschrieben wird, erhält man ein Gemisch aus isomeren 5-, 4- und 3-Formylvaleriansäureestern. Für die weitere Verwendung wird jedoch häufig nur eines der Isomeren benötigt, während die übrigen Isomeren als Nebenprodukte verbleiben. Wenn z.B. 5-Formylvaleriansäureester das gewünschte Endprodukt ist, so verbleiben 4- und 3-Formylvaleriansäureester als nichtverwertbare Nebenprodukte.

Aus Journal of American Chemical Society, Band 90 (1968), Seiten 94-98, war bereits bekannt, daß man unverzweigte Aldehyde in Gegenwart von metallischem Palladium oder Platin auf Trägern zu Olefinen umsetzt, die ein Kohlenstoffatom weniger als der Ausgangsaldehyd enthalten. Ausgehend von n-Decanal wurde bei 190 bis 195°C in Gegenwart von Palladium auf Aktivkohle ein Gemisch aus 3 isomeren Nonenen sowie 27 % Nonan erhalten. Die Spaltung von $\beta$-Phenylpropionaldehyd lieferte dagegen 37 % Ethylbenzol und nur Spuren Styrol.

Auch bei der Spaltung von unverzweigten Aldehyden in Gegenwart von Rhodiumkomplexverbindungen wie Chlorotris(triphenylphosphin)rhodium werden überwiegend gesättigte Kohlenwasserstoffe gebildet. So erhält man ausgehend von n-Heptanal 86 % Hexan, wie aus Journal of American Chemical Society, Band 90 (1968), Seiten 99-107, hervorgeht.

In der US-Patentschrift 4 517 400 wird die Umsetzung von Gemischen aus unverzweigten und verzweigten Aldehyden an Übergangsmetall-Trägerkatalysatoren beschrieben. In Gegenwart von Trägerkatalysatoren, die katalytisch aktive Metalle wie Platin, Palladium, Rhodium, Kupfer oder Zink enthalten, werden hierbei fast ausschließlich die im Aldehydgemisch enthaltenen geradkettigen Aldehyde zu Olefinen gespalten, während die verzweigten Aldehyde praktisch nicht reagieren.

Die Spaltung von Isobutyraldehyd bei Temperaturen von 200 bis 400°C in der Gasphase an Rhodium- und/oder Platin-Trägerkatalysatoren ist bereits aus der deutschen Patentschrift 19 17 244 bekannt. Unterhalb von 250°C sinkt der Umsatz jedoch beträchtlich ab, so daß der eingesetzte Isobutyraldehyd zum großen Teil unverändert wiedergewonnen wird.

Da aus der europäischen Patentanmeldung 81 090 bekannt war, daß 4-Formylcarbonsäureester bei Temperaturen zwischen 150 und 600°C zu 3,4-Dihydro-2-pyronen cyclisieren, war davon auszugehen, daß Formylvaleriansäureester analog cyclisiert werden.

Es war deshalb die technische Aufgabe gestellt, ein Verfahren zur Herstellung von Pentensäureestern aus 3- und/oder 4-Formylvaleriansäureestern zur Verfügung zu stellen, bei dem eine Cyclisierung der Formylvaleriansäureester vermieden wird, Pentensäureester mit hohem Umsatz und guter Ausbeute erhalten werden und zudem wenig gesättigte Carbonsäureester anfallen.

Diese Aufgabe wird gelöst in einem Verfahren zur Herstellung von Pentensäureestern, dadurch gekennzeichnet, daß man 3- und/oder 4-Formylvaleriansäureester in Gegenwart von Katalysatoren, die mindestens ein Element der VIII. Nebengruppe enthalten, bei einer Temperatur von 50 bis 400°C behandelt.

Das neue Verfahren liefert Pentensäureester mit hohem Umsatz und hoher Selektivität. Die Hydrierung zu gesättigten Carbonsäureestern wird weitgehend vermieden. Die Cyclisierung zu Dihydropyronen kann praktisch vollständig ausgeschlossen werden.

Als Einsatzstoffe dienen 4- und 3-Formylvaleriansäureester einzeln und im Gemisch, die sich vorzugsweise von Alkanolen mit 1 bis 12 Kohlenstoffatomen oder Cycloalkanolen mit 5 bis 8 Kohlenstoffatomen, ableiten. Besonders bevorzugt sind Formylvaleriansäureester von niedrigen Alkanolen, insbesondere solchen mit 1 bis 6 Kohlenstoffatomen, insbesondere Methanol. Beispielsweise seien genannt Methyl-, Ethyl-, Propyl-, Isopropyl-, Butyl-, Pentyl-, Hexyl-, Nonyl-, Dodecyl- oder Cyclohexylester von jeweils 4- oder 3-Formylvaleriansäure. Besondere technische Bedeutung haben Gemische aus 4- und 3-Formylvaleriansäureestern, die noch geringe Mengen an 5-Formylvaleriansäureestern enthalten können. Typische Gemische enthalten beispielsweise 60 bis 75 Gew.% 4-Formylvaleriansäureester, 25 bis 35 Gew.% 3-Formylvaleriansäureester und bis zu 5 Gew.% 5-Formylvaleriansäureester.

Erfindungsgemäß verwendet man Katalysatoren, die mindestens ein Element der VIII. Nebengruppe des Periodischen Systems enthalten.

Es ist möglich, Komplexverbindungen von Edelmetallen der VIII. Nebengruppe, insbesondere Ruthenium oder Rhodium als homogene Katalysatoren zu verwenden. Insbesondere sind Halogene wie Chlor oder Brom und Phosphine oder Phosphite enthaltende Rutheinium- oder Rhodiumkomplexe geeignet, die zusätzlich als Liganden Kohlenmonoxid enthalten können. Besonders bevorzugt werden als Modifizierungsmittel tertiäre organische Phosphine verwendet. Bevorzugte Substituenten solcher Phosphine sind Alkylreste mit bis zu 18 Kohlenstoffatomen, Cycloalkylreste mit 5 bis 12 Kohlenstoffatomen, Aralkylreste mit 7 bis 10 Kohlenstoffatomen oder Arylreste mit 6 bis 10 Kohlenstoffatomen, insbesondere Phenylreste. Die Reste können gleich oder verschieden sein. Beispiele für geeignete Komplexverbindungen sind RhCl[P(C$_6$H$_5$)$_3$]$_3$, Ru$_2$Cl$_3$[(P(C$_6$H$_5$)(C$_2$H$_5$)$_2$)$_6$]Cl, RhBr(CO)[P(C$_6$H$_5$)$_3$]$_2$, HRuCl(CO)[P(C$_6$H$_5$)$_3$]$_3$, RhCl(CO)[P(C$_6$H$_5$)$_3$]$_2$.

Bevorzugt werden Trägerkatalysatoren verwendet, die mindestens eines der Elemente Palladium, Platin, Ruthenium, Rhodium, Osmium, Iridium, Eisen, Kobalt oder Nickel, insbesondere Edelemetalle enthalten. Vorteilhaft sind auch Trägerkatalysatoren, die mindestens zwei Edelmetalle, ausgewählt aus der Gruppe Ruthenium, Rhodium, Palladium, Platin, Iridium oder Osmium enthalten. Andere bevorzugte Trägerkatalysatoren enthalten mindestens ein Metall der vorgenannten Edelmetalle der VIII. Nebengruppe des Periodischen Systems und zusätzlich mindestens ein Metall, ausgewählt aus der Gruppe Eisen, Kobalt und Nickel.

Die Trägerkatalysatoren haben vorteilhaft eine Gehalt an aktiven Metallen der VIII. Nebengruppe des Periodischen Systems von 0,01 bis 10, vorzugsweise 0,05 bis 5, insbesondere von 0,05 bis 1 Gew.%, bezogen auf die Summe aus Träger und katalytisch aktiven Metallen, berechnet als Metall. Als Träger werden vorteilhaft Aluminiumoxid, Siliciumdioxid, Titandioxid, Zinkoxid, Lanthanoxid, Zirkondioxid, Bariumsulfat, Aluminiumsilikate oder Gemische derselben verwendet.

Besonders vorteilhafte Trägerkatalysatoren enthalten zusätzlich noch mindestens ein Element der I. bis VII. Nebengruppe und/oder Elemente der seltenen Erden, z.B. Zink, Kupfer, Silber, Lanthan, Titan, Vanadium, Chrom, Molybdän, Wolfram, Mangan, Rhenium, Cer, Neodym oder Praseodym, vorteilhaft in einer Menge von 0,05 bis 2 Gew.%, berechnet als Metall und bezogen auf das Gesamtgewicht des Katalysators (Träger und katalytisch aktive Metalle).

Bewährt haben sich z.B. Tränkkatalysatoren, bei denen die katalytisch wirksamen Metalle an der Oberfläche des Trägers angereichert sind. Derartige Katalysatoren werden in an sich bekannter Weise durch Tränken vorgeformter Träger wie Pellets, Kugeln oder Stränge mit einer wäßrigen Lösung der Metallsalze, die beim Erhitzen in deren Oxide übergehen, z.B. der Nitrate, die dann getrocknet, calciniert und direkt bzw. gegebenenfalls nach Reduktion mit Wasserstoff oder anderen Reduktionsmitteln eingesetzt werden können.

Die bevorzugt verwendeten Trägerkatalysatoren zeigen über einen längeren Zeitraum hohe Aktivität. Verbrauchte Katalysatoren lassen sich durch Behandlung mit molekularen Sauerstoff enthaltenden Gasen, z.B. Luft, bei einer Temperatur von 350 bis 500°C und anschließende Reduktion regenerieren.

Bei der Spaltung der Formylvaleriansäureester hält man eine Temperatur von 50 bis 400°C, vorteilhaft 60 bis 350°C, vorzugsweise 100 bis 280°C, insbesondere 120 bis 200°C ein. Im allgemeinen wird die Spaltung unter Atmosphärendruck durchgeführt. Es ist jedoch auch möglich, verminderten Druck oder erhöhten Druck, Zweckmäßig im Bereich von 10 mbar bis 20 bar, anzuwenden. Im allgemeinen hält man eine Katalysatorbelastung von 0,01 bis 40, vorzugsweise 0,1 bis 20 kg Formylvaleriansäureester je kg Katalysator und Stunde ein.

Es kann zweckmäßig sein, die Spaltung der Formylvaleriansäureester unter zusätzlicher Mitverwendung von Verdünnungsmitteln durchzuführen. Geeignete Verdünnungsmittel sind Wasser, Alkohole wie Methanol, Ethanol, Butanol oder Cyclohexanol, ferner Ether wie Dioxan oder Tetrahydrofuran sowie chlorierte Kohlenwasserstoffe wie Methylenchlorid, Chloroform, 1,2-Dichlormethan oder weiterhin aliphatische, cycloaliphatische oder aromatische Kohlenwasserstoffe wie Benzol, Toluol, Cyclohexan, Paraffine, ferner Ester wie Essigsäureester oder Propionsäureester. Vorteilhaft wird der Alkohol verwendet, der dem in den Formylvaleriansäureestern gebundenen Alkohol entspricht. Edukt und Produkt sind so durch eine ausreichende Siedepunktdifferenz destillativ gut trennbar. Es hat sich bewährt, wenn das Molverhältnis von Formylvaleriansäureestern zu Verdünnungsmitteln 1:0,1 bis 1:50, insbesondere 1:0,5 bis 1:20 beträgt. Besonders bevorzugte Verdünnungsmittel sind Wasser und Alkanole mit 1 bis 6 Kohlenstoffatomen oder Gemische derselben, insbesondere Methanol.

Vorteilhaft führt man die Spaltung unter Mitverwendung von molekularem Sauerstoff oder molekularen Sauerstoff enthaltenden Gasen, die neben Sauerstoff Inertgase wie Stickstoff, Kohlendioxid, Argon oder Wasserdampf enthalten, durch. Vorzugsweise wendet man ein Molverhältnis von Formylvaleriansäureester zu molekularem Sauerstoff von 1:0,05 bis 1:3, insbesondere 1:0,2 bis 1:1,5, z.B. 1:0,5 bis 1,25, an. Hierdurch wird die Lebensdauer des Katalysators erhöht und insbesondere die Ausbeute an Pentenestern gesteigert. Die Mitverwendung von molekularem Sauerstoff war nicht angezeigt, da 5-Formylvaleriansäuremethylester schon bei 50°C durch molekularen Sauerstoff mit 96 %iger Ausbeute zu Adipinsäuremonomethylester oxidiert wird, wie aus der europäischen Patentschrift 131 860 bekannt ist und somit zu erwarten war, daß 4- und 3-Formylvaleriansäureester analog zu 2-Methylglutarsäuremonomethylester und 3-Ethylbernsteinsäuremonomethylester oxidiert werden.

Die Umsetzung kann diskontinuierlich oder kontinuierlich, als Festbettreaktion mit fest angeordneten Katalysatoren, beispielsweise in Sumpf-oder Rieselfahrweise, in der Flüssig- oder Gasphase oder als Wirbelbettreaktion mit in auf- und abwirbelnder Bewegung befindlichen Katalysatoren in der Gasphase oder in der Flüssigphase mit löslichen Katalysatoren oder suspendierten Trägerkatalysatoren durchgeführt werden.

Eine bevorzugte Ausführungsform in der Flüssigphase führt man z.B. so durch, daß man Formylvaleriansäureester und gegebenenfalls Verdünnungsmittel mit Sauerstoff enthaltenden Gasen bei einer Temperatur unterhalb des Siedepunkts des Formylvaleriansäureesters über einen festen Katalysator leitet oder in Gegenwart eines suspendierten, festen Katalysators oder eines gelösten Homogenkatalysators erhitzt. Das flüssige Reaktionsprodukt wird anschließend nach Abtrennung der Katalysatoren durch Destillation in Pentensäureester sowie gegebenenfalls Verdünnungsmittel und nicht umgesetzte Formylvaleriansäureester, die zurückgeführt werden können, getrennt.

Eine bevorzugte Ausführungsform des erfindungsgemäßen Verfahrens in der Gasphase führt man z.B. durch, indem man ein Gemisch aus Formylvaleriansäureester und gegebenenfalls Verdünnungsmittel verdampft und dann zusammen mit Luft und zweckmäßig zusätzlich einem Trägergas wie Stickstoff, Kohlendioxid oder Argon bei der vorgenannten Temperatur gasförmig in eine fest angeordnete oder eine in auf- und abwirbelnder Bewegung befindliche Katalysatorschicht einleitet. Der Reaktionsaustrag wird kondensiert und anschließend durch fraktionierende Destillation aufgetrennt. Nicht umgesetzte Formylvaleriansäureester werden zweckmäßig zurückgeführt. Das erhaltene Gemisch aus 4-, 3- und 2-Pentensäureestern ist unmittelbar für die Hydroformylierung zu 5-Formylvaleriansäureestern verwendbar.

Das Verfahren nach der Erfindung sei an folgenden Beispielen veranschaulicht.

Beispiel 1

Pro Stunde wurden 10 ml eines Gemisches aus 4-Formylvaleriansäuremethylester (4-FVSE), Tetrahydrofuran und Wasser (23 Gew.% 4-FVSE, 10 Gew.% Wasser, Rest Tetrahydrofuran) in einen Verdampfer gepumpt und von dort zusammen mit 3 l Stickstoff bei 250°C über 10 g Katalysator geleitet. Die gasförmigen Reaktionsausträge wurden in Kühlfallen kondensiert, gewogen und gaschromatograpahisch analysiert. In der Tabelle 1 ist die Zusammensetzung der Reaktionsausträge nach jeweils 4 h Versuchsdauer angegeben (PSE = Pentensäuremethylester).

Tabelle 1

| Nr. | Katalysator | PSE[1] mol-% | Umsatz % | PSE-Selekt. % |
|-----|-------------|--------------|----------|---------------|
| 1 | 1 % Pt auf $SiO_2$ | 32 | 51 | 63 |
| 2 | 0,5 % Ru/0,5 % Rh auf $SiO_2$ | 61 | 88 | 69 |
| 3 | 0,5 % Rh/0,5 % Pt auf $SiO_2$ | 55 | 96 | 57 |
| 4 | 0,5 % Rh/0,5 % Ru/0,5 % Pt auf $SiO_2$ | 71 | 99 | 71 |

[1] Gemisch isomerer Pentensäuremethylester

Vergleichsbeispiel 1

Wurde Beispiel 1 mit 10 g $SiO_2$ als Katalysator weiderholt, das für die Herstellung der in Tabelle 1 genannten Katalysatoren verwendet worden war, jedoch ohne Zusatz von Platin, so einthielt der Reaktionsaustrag nach 4 Stunden Reaktionszeit laut gaschromatographischer Analyse (Fl.%) 70,8 % 4-FVSE und 25,9 % 5-Methyl-3,4-dihydro-2-pyron.

Beispiel 2

Pro Stunde wurden 10 ml eines Gemisches aus 4-FVSE, Methanol und Wasser (30 Gew.% 4-FVSE, 10 Gew.% Wasser, Rest Methanol) in einen Verdampfer gepumpt und von dort zusammen mit 3 l Luft bei 250°C über 10 g Katalysator geleitet. Die gasförmigen Reaktionsausträge wurden in Kühlfallen kondensiert, gewogen und gaschromatographisch analysiert. Der verwendete Katalysator und die erzielten Ergebnisse sind aus Tabelle 2 zu entnehmen.

Tabelle 2

| Nr. | Katalysator | PSE mol-% | Umsatz % | PSE-Selekt. % |
|-----|-------------|-----------|----------|---------------|
| 1 | 1 % Pt auf SiO$_2$ | 26 | 52 | 49 |
| 2 | 0,5 % Ru/0,9 % Rh auf SiO$_2$ | 67 | 96 | 70 |
| 3 | 0,5 % Rh/0,5 % Pt auf SiO$_2$ | 44 | 99 | 44 |
| 4 | 0,5 % Rh/0,5 % Ru/0,5 % Pt auf SiO$_2$ | 72 | 94 | 77 |
| 5 | 0,5 % Rh/0,5 % Ru/0,5 % Ag auf SiO$_2$ | 58 | 98 | 59 |
| 6 | 0,5 % Rh/0,5 % Ru/0,5 % Fe auf SiO$_2$ | 52 | 91 | 57 |
| 7 | 0,5 % Rh/0,5 % Ru/0,5 % Cr auf SiO$_2$ | 35 | 98 | 36 |

Beispiel 3

Pro Stunde wurden 10 ml eines Gemisches aus 4-FVSE, Methanol und Wasser (30 Gew.% 4-FVSE, 10 Gew.% Wasser, Rest Methanol) in einen Verdampfer gepumpt und von dort zusammen mit 3 l Luft bei 250°C über 10 g Katalysator (0,5 % Ru, 0,5 % Rh und 0,5 % Pt auf Siliciumdioxid) geleitet. Die gasförmigen Reaktionsausträge wurden in Kühlfallen kondensiert, gewogen und gaschromatographisch analysiert. In der Tabelle 3 ist die Zusammensetzung der Reaktionsausträge nach der jeweiligen Versuchsdauer angegeben.

Tabelle 3

| Nr. | Versuchsdauer h | PSE mol-% | Umsatz % | PSE-Selektivität % |
|-----|-----------------|-----------|----------|--------------------|
| 1 | 10 | 60 | 99 | 67 |
| 2 | 34 | 66 | 99 | 67 |
| 3 | 58 | 70 | 95 | 74 |
| 4 | 130 | 72 | 94 | 77 |
| 5 | 198 | 64 | 84 | 76 |
| 6 | 300 | 65 | 84 | 77 |

Nach 300 h Versuchsdauer wurden die vereinigten Reaktionsausträge fraktionierend destilliert:
Austrag: 428 g Pentensäuremethylester/Valeriansäuremethylester-Gemisch (80 % Pentenester (25 % 4-, 70 % 3- und 5 % 2-trans-Pentensäuremethylester)/20 % Valerianester) = 60 % d. Th. Ausbeute an Pentenester und 15 % d.Th. Valerianester

Beispiel 4

Pro Stunde wurden 45 ml einer 37,5 gew.%igen 3-FVSE-Lösung in Methanol in einen Verdampfer gepumpt und von dort zusammen mit 12 l Luft bei 250°C über 30 g Katalysator (0,5 % Ru und 0,85 % Rh auf Siliciumdioxid) geleitet. Die gasförmigen Reaktionsausträge wurden über 6 h Versuchsdauer in Kühlfallen kondensiert, gewogen und gaschromatographisch analysiert. Das Ergebnis ist wie folgt:

| PSE [mol%] | VSE [mol%] | Umsatz [%] | PSE-Selektivität [%] |
|------------|------------|------------|----------------------|
| 60 | 15 | 85 | 71 |

Beispiel 5

Pro Stunde wurden 45 ml einer 37,5 gew.%igen FVSE-Lösung in Methanol (63 % 4-FVSE, 32 % 3-FVSE) in einen Verdampfer gepumpt und von dort zusammen mit 12 l Luft bei 250°C über 30 g Katalysator (0,5 % Ru und 0,85 % Rh auf Siliciumdioxid) geleitet. Die gasförmigen Reaktionsausträge wurden über 100 h Versuchsdauer in Kühlfallen kondensiert, gewogen und gaschromatographisch analy-

5

siert. Das Ergebnis ist wie folgt:

| PSE [mol%] | VSE [mol%] | Umsatz [%] | PSE-Selektivität [%] |
|---|---|---|---|
| 62 | 13 | 82 | 76 |

Beispiel 6

In einem 1 l-Reaktionskolben mit einer Destillationsvorrichtung für die Reaktionsprodukte wurden als Lösungsmittel 150 g hochsiedende Aldolisierungsprodukte der Formylvaleriansäuremethylester vorgelegt, die 10 g Chlorocarbonyl-bis(triphenylphosphin)-rhodium-Komplex (ClRh(CO)(PPh$_3$)$_2$) enthielten.

Zu dieser Katalysatorlösung wurden bei 230 bis 240°C pro Stunde 50 g eines Formylvaleriansäureester-Gemisches (69 % 4-FVSE, 29 % 3-FVSE, 2 % 5-FVSE) zugegeben.

Die Reaktionsprodukte wurden kontinuierlich abdestilliert. Pro Stunde wurden 47 g Destillat erhalten, die nach gaschromatographischer Analyse 10,1 Gew.% Valeriansäuremethylester und 50,2 % Pentensäuremethylester (Isomerengemisch) enthielten. Dies entspricht einer Ausbeute von 60 % an Pentensäuremethylester (bez. auf eingesetzten Formylvalerianester).

Beispiel 7

Pro Stunde wurden 30 g 96,6 %iger 4-Formylvaleriansäuremethylester zusammen mit 24 l/h N$_2$ und 6 l/h Luft bei 130°C über 20 g Katalysator (0,75 % Ni, 0,25 % Pd auf Kieselsäure) geleitet. Aus 186,1 g Ausgangsmaterial erhielt man 162 g Reaktionsaustrag. Die Destillation des Austrags erbrachte 66,8 g eines Produktes mit einem Siedebereich von 93 bis 145°C, welches zu 82 % aus PSE-Isomeren bestand. Der Destillationssumpf von 93 g enthielt 77,7 % FVSE und 9,6 % PSE. Daraus ergab sich ein Umsatz von 62 % und eine Selektivität von 77 %.

Beispiele 8 bis 18

Käuflicher Katalysatorträger wurde mit einer Metallsalzlösung berechneten Gehalts an Metallionen 3 Stunden bis zur Sättigung getränkt. Dann wurde im Vakuum getrocknet und anschließend bei 300 bis 400°C unter Stickstoff calciniert. Die Katalysatoren wurden dann ohne weitere Veränderung für die Umsetzungen verwendet.

Pro Stunde wurden 30 g 4-Formylvaleriansäuremethylester und 30 l Gas der angegebenen Zusammensetzung bei der entsprechenden Temperatur über 10 g Katalysator geleitet. Der kondensierte flüssige Reaktionsaustrag wurde gaschromatographisch analysiert und daraus Umsatz und Selektivität berechnet. Die Einzelheiten sind aus Tabelle 4 zu entnehmen.

Tabelle 4

| Bsp. | Katalysator | | Gas N$_2$/Luft [Vol.-Teile] | Temp. [$^0$C] | PSE [Gew.%] | Umsatz [%] | Selekt.PSE [%] |
|---|---|---|---|---|---|---|---|
| 8 | 0,25 % Pd 0,75 % Ni | SiO$_2$ | 4/1 | 130 | 76 | 96 | 76 |
| 9 | 0,75 % Pd 0,04 % Mn | SiO$_2$ | 2/3 | 180 | 75 | 87 | 90 |
| 10 | 0,75 % Pd 0,25 % Ag | SiO$_2$ | 4/1 | 180 | 47 | 79 | 55 |
| 11 | 0,75 % Pd 0,1 % Re | SiO$_2$ | 3/2 | 180 | 73 | 86 | 91 |
| 12 | 0,75 % Pd 0,1 % V | SiO$_2$ | 4/1 | 180 | 46 | 74 | 70 |
| 13 | 0,75 % Pd 0,1 % Ce | SiO$_2$ | 3/2 | 180 | 72 | 89 | 85 |
| 14 | 0,5 % Pd 0,5 % Ru | SiO$_2$ | 4/1 | 150 | 6 | 34 | 22 |

Tabelle 4 (Fortsetzung)

| Bsp. | Katalysator | | Gas N$_2$/Luft [Vol.-Teile] | Temp. [$^0$C] | PSE [Gew.%] | Umsatz [%] | Selekt.PSE [%] |
|---|---|---|---|---|---|---|---|
| 15 | 0,75 % Pd 0,1 % Zn | SiO$_2$ | 3/2 | 180 | 70 | 86 | 89 |
| 16 | 0,75 % Pd 0,25 % Ag | ZrO$_2$ | 4/1 | 180 | 11 | 26 | 51 |
| 17 | 0,75 % Pd 0,25 % Ag | TiO$_2$ | 4/1 | 180 | 46 | 62 | 76 |
| 18 | 1,1 % Pd | BaSO$_4$ | 4/1 | 190 | 17 | 25 | 81 |

Vergleichsbeispiel 2

In eine Blasensäule, die 217 g 4-Formylvaleriansäuremethylester enthielt, wurden bei 50°C pro Stunde 5 Liter Sauerstoff von unten und 40 Liter Stickstoff oberhalb der Flüssigphase eingeleitet. Nach 7 Stunden Reaktionszeit wurde mit Stickstoff gespült. Durch fraktionierende Destillation des Reaktionsaustrages wurden 194 g 2-Methylglutarsäure-5-methylester (81 % d.Th.) erhalten.

**Patentansprüche**

1. Verfahren zur Herstellung von Pentensäureestern, dadurch gekennzeichnet, daß man 3- und/oder 4-Formylvaleriansäureester in Gegenwart von Katalysatoren, die mindestens ein Element der VIII. Nebengruppe des Periodischen Systems enthalten, auf eine Temperatur von 50 bis 400°C erhitzt.

2. Verfahren nach Anspruch 1, dadurch gekennzeichnet, daß man 3- und/oder 4-Formylvaleriansäuremethylester verwendet.

3. Verfahren nach den Ansprüchen 1 und 2, dadurch gekennzeichnet, daß man Trägerkatalysatoren verwendet, die 0,01 bis 10 Gew.% mindestens eines Metalls der VIII. Nebengruppe des Periodischen Systems, berechnet als Metall, bezogen auf die Summe von Träger und aktiven Metallen, enthalten.

4. Verfahren nach den Ansprüchen 1 bis 3, dadurch gekennzeichnet, daß die Katalysatoren mindestens zwei Metalle, ausgewählt aus der Gruppe Ruthenium, Rhodium, Palladium, Platin, Iridium und Osmium, enthalten.

5. Verfahren nach den Ansprüchen 1 bis 4, dadurch gekennzeichnet, daß die Katalysatoren mindestens ein Edelmetall der VIII. Nebengruppe des Periodischen Systems und zusätzlich mindestens ein Metall, ausgewählt aus der Gruppe Eisen, Kobalt und Nickel, enthalten.

6. Verfahren nach den Ansprüchen 1 bis 5, dadurch gekennzeichnet, daß die Katalysatoren zusätzlich einen Gehalt mindestens eines Elements der I. bis VII. Nebengruppe des Periodischen Systems haben.

7. Verfahren nach den Ansprüchen 1 und 2, dadurch gekennzeichnet, daß man als Katalysatoren Komplexverbindungen von Ruthenium oder Rhodium verwendet.

8. Verfahren nach den Ansprüchen 1 bis 7, dadurch gekennzeichnet, daß man eine Temperatur von 60 bis 350°C einhält.

9. Verfahren nach den Ansprüchen 1 bis 8, dadurch gekennzeichnet, daß man molekularen Sauerstoff oder molekularen Sauerstoff enthaltende Gase mitverwendet.

10. Verfahren nach den Ansprüchen 1 bis 9, dadurch gekennzeichnet, daß man ein Molverhältnis von 3- und/oder 4-Formylvaleriansäureester zu molekularem Sauerstoff von 1:0,05 bis 1:3 einhält.

11. Verfahren nach den Ansprüchen 1 bis 10, dadurch gekennzeichnet, daß man Verdünnungsmittel mitverwendet.

12. Verfahren nach den Ansprüchen 1 bis 11, dadurch gekennzeichnet, daß man als Verdünnungsmittel Alkohole verwendet, die den in den Formylestern gebundenen Alkoholen entsprechen.

13. Verfahren nach den Ansprüchen 1 bis 12, dadurch gekennzeichnet, daß man ein Molverhältnis von Formylvaleriansäureester zu Verdünnungsmittel von 1:0,1 bis 1:50 einhält.

**Claims**

1. A process for the preparation of a pentenoic ester, wherein 3- and/or 4-formylvaleric esters are heated to 50-400°C in the presence of a catalyst which contains one or more elements of subgroup VIII of the Periodic Table.

2. A process as claimed in claim 1, wherein methyl 3-formylvalerate and/or methyl 4-formylvalerate are used.

3. A process as claimed in claim 1 or 2, wherein a supported catalyst which contains from 0.01 to 10% by weight, calculated as metal and based on the sum of the carrier and active metals, of one or more metals of subgroup VIII of the Periodic Table is used.

4. A process as claimed in any of claims 1 to 3, wherein the catalyst contains two or more metals selected from the group consisting of ruthenium, rhodium, palladium, platinum, iridium and osmium.

5. A process as claimed in any of claims 1 to 4, wherein the catalyst contains one or more noble metals of subgroup VIII of the Periodic Table and in addition one or more metals selected from the group consisting of iron, cobalt and nickel.

6. A process as claimed in any of claims 1 to 5, wherein the catalyst additionally contains one or more elements of subgroups I to VII of the Periodic Table.

7. A process as claimed in claim 1 or 2, wherein the catalyst used is a complex of ruthenium or rhodium.

8. A process as claimed in any of claims 1 to 7, wherein a temperature of from 60 to 350°C is maintained.

9. A process as claimed in any of claims 1 to 8, wherein molecular oxygen or a gas containing molecular oxygen is concomitantly used.

10. A process as claimed in any of claims 1 to 9, wherein a molar ratio of 3- and/or 4-formylvaleric esters to molecular oxygen of from 1 : 0.05 to 1 : 3 is maintained.

11. A process as claimed in any of claims 1 to 10, wherein a diluent is present.

12. A process as claimed in any of claims 1 to 11, wherein the diluent used is an alcohol which corresponds to the alcohol bonded in the formyl esters.

13. A process as claimed in any of claims 1 to 12, wherein a molar ratio of formylvaleric esters to diluent of from 1 : 0.1 to 1 : 50 is maintained.

**Revendications**

1. Procédé de préparation d'esters d'acide penténoïque, caractérisé en ce qu'on chauffe à une température de 50 à 400°C un ester d'acide 3- et/ou 4-formylvalérique en présence de catalyseurs qui contiennent au moins un élément du groupe VIII de la classification périodique.

2. Procédé selon la revendication 1, caractérisé en ce qu'on utilise le 3- et/ou 4-formylvalérate de méthyle.

3. Procédé selon les revendications 1 et 2, caractérisé en ce qu'on utilise des catalyseurs supportés qui contiennent de 0,01 à 10% en poids d'au moins un métal du groupe VIII de la classification périodique, calculé en métal, par rapport à la somme du support et des métaux actifs.

4. Procédé selon les revendications 1 à 3, caractérisé en ce que les catalyseurs contiennent au moins deux métaux, choisis dans le groupe comprenant le ruthénium, le rhodium, le palladium, le platine, l'iridium et l'osmium.

5. Procédé selon les revendications 1 à 4, caractérisé en ce que les catalyseurs contiennent un métal noble du groupe VIII de la classification périodique et au moins un métal choisi dans le groupe comprenant le fer, le cobalt et le nickel.

6. Procédé selon les revendications 1 à 5, caractérisé en ce que les catalyseurs ont également une teneur

EP 0 295 548 B1

en au moins un élément des groupes Ib à VIIb de la classification périodique.

7. Procédé selon les revendications 1 et 2, caractérisé en ce qu'on utilise comme catalyseurs des composés complexes du ruthénium ou du du rhodium.

8. Procédé selon les revendications 1 à 7, caractérisé en ce qu'on maintient une température de 60 à 350°C.

9. Procédé selon les revendications 1 à 8, caractérisé en ce qu'on utilise également de l'oxygène moléculaire ou des gaz contenant de l'oxygène moléculaire.

10. Procédé selon les revendications 1 à 9, caractérisé en ce qu'on utilise un rapport molaire de l'ester d'acide 3- et/ou 4-formylvalérique à l'oxygène moléculaire de 1:0,05 à 1:3.

11. Procédé selon les revendications 1 à 10, caractérisé en ce qu'on utilise également des diluants.

12. Procédé selon les revendications 1 à 11, caractérisé en ce qu'on utilise comme diluant des alcools qui correspondent aux alcools liés dans les formylvalérates.

13. Procédé selon les revendications 1 à 12, caractérisé en ce qu'on utilise un rapport molaire du formylvalérate au diluant 1:0,1 à 1:50.

10